# EUROPEAN PATENT APPLICATION

(11) **EP 2 574 355 A1**
(43) Date of publication of application: **03.04.2013**
(21) Application number: 11182974.3
(22) Date of filing: 27.09.2011
(51) Int. Cl.: A61M 5/00, A61M 5/32

(54) **Package for a medicament delivery device**

(71) Applicant: Sanofi-Aventis Deutschland GmbH, 65929 Frankfurt (DE)
(72) Inventor: The designation of the inventor has not yet been filed

(57) **Abstract**

Described is a package for a medicament delivery device having a needle shield covering a needle. The package (1) comprises a base element (2) including a storage compartment (3) adapted to contain the delivery device (D), and a needle cap remover (4) formed on the base element (2). The needle cap remover (4) adapted to engage the needle shield (D.3).

## Description

### Technical Field

The invention relates to a package for a medicament delivery device, e.g. a syringe, a pen injector, an auto-injector, etc. The packaging may also be applied to safety needle assemblies and other types of medical devices having needles (e.g., infusion systems).

### Background of the Invention

A conventional medicament device may include a device covering a needle. For example, the device may be a rigid needle shield ("RNS") which maintains sterility of the needle and prevents needlestick injury. However, when removing the RNS, in addition to applying an axial force to remove the RNS from the needle, a rotational force and/or an angled force may be applied to the RNS. If the rotation force is applied, a distal tip of the needle may cut away pieces of the RNS which could potentially become lodged in the needle. If the angled force is applied (e.g., if the RNS is pulled at an angle to its axis), the needle may be bent. In either case, an undersirable effect may occur during removal of the RNS. Thus, there is a need for an improved mechanism for removing the RNS.

### Summary of the Invention

It is an object of the present invention to provide a package for storing a medicament delivery device that is safe to handle, minimizes the risk of accidental needle stick injuries, and prevents damage to the needle.

In an exemplary embodiment, a package for a medicament delivery device having a needle shield covering a needle comprises a base element including a storage compartment adapted to contain the delivery device, and a needle cap remover formed on the base element. The needle cap remover is adapted to engage the needle shield. In an exemplary embodiment, the needle cap remover is an upstanding projection. In another exemplary embodiment, the needle cap remover is in the storage compartment.

In an exemplary embodiment, the needle cap remover comprises at least a retainer adapted to retain the needle shield. The retainer includes resilient arms adapted to deflect when the needle shield is inserted into the retainer. The resilient arms frictionally engage the needle shield. The retainer is a recess adapted to engage a latch on the needle shield.

In an exemplary embodiment, a seal covers the storage compartment.

Further scope of applicability of the present invention will become apparent from the detailed description given hereinafter. However, it should be understood that the detailed description and specific examples, while indicating preferred embodiments of the invention, are given by way of illustration only, since various changes and modifications within the spirit and scope of the invention will become apparent to those skilled in the art from this detailed description.

### Brief Description of the Drawings

The present invention will become more fully understood from the detailed description given hereinbelow and the accompanying drawings which are given by way of illustration only, and thus, are not limitive of the present invention, and wherein:
- Figure 1: shows an isometric view of an exemplary embodiment of a package for a medicament delivery device.
- Figures 2A, 2B: show an isometric view of an exemplary embodiment of a package during removing of a seal.
- Figure 3: shows a lateral view of an exemplary embodiment of a package during removal of a cap and needle shield.
- Figure 4: shows an isometric view of an exemplary embodiment of a package after removal of cap and needle shield.
- Figures 5 to 7: show sectional views of an exemplary embodiment of a package with a needle shield remover during removal of the needle shield.
- Figure 8: shows a sectional view another exemplary embodiment of a package with a needle shield remover.
- Figures 9 to 11: show sectional views of exemplary embodiments of packages with integrated needle cap removers according to alternative embodiments.
Corresponding parts are marked with the same reference symbols in all figures.

### Detailed Description

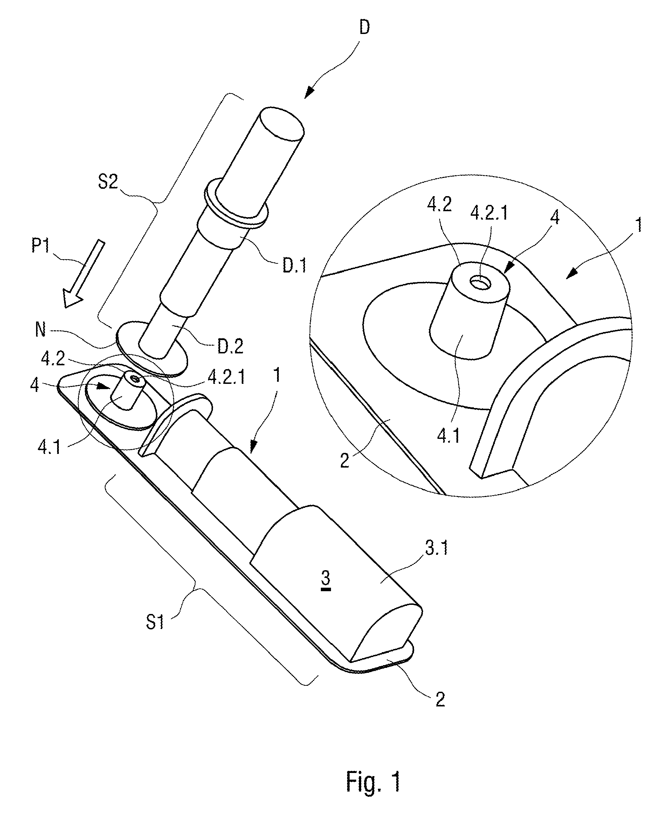

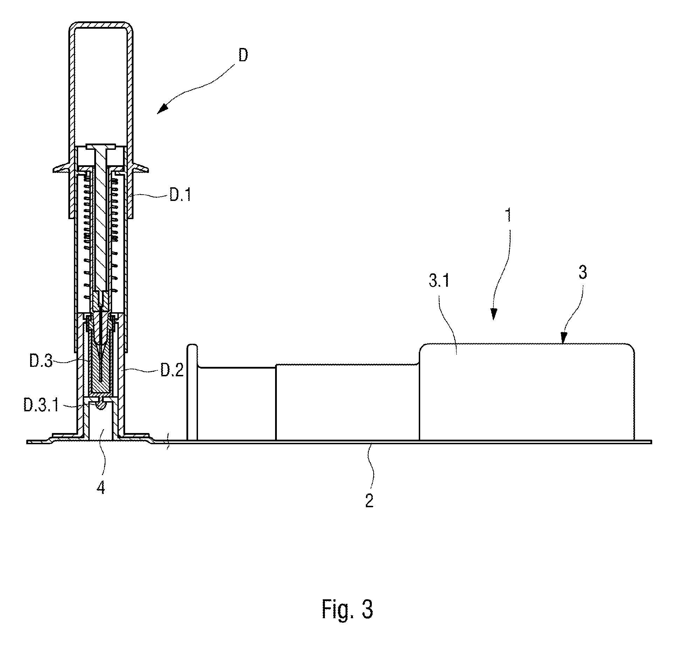

Figure 1 shows an isometric view of an exemplary embodiment of a package 1 for a medicament delivery device D according to the invention. According to exemplary embodiments of the invention, the delivery device D may be arranged as a syringe, a dental syringe, an auto-injector, a pen injector or a similar device suitable for delivering a drug or a medicament to a patient. In the exemplary embodiment shown in Figure 1, the delivery device D comprises a housing D.1 adapted to contain a (not shown) prefilled syringe or a cartridge with a mounted or integrally formed needle assembly with an attached needle and a cap D.2 which covers the needle before and after an injection. The housing D.1 may comprise one or more substantially cylindrical sleeves. In an exemplary embodiment, the cap D.2 is adapted to be axially slidably mounted to the housing D.1 of the delivery device D. Furthermore, the needle is usually covered by a needle shield D.3, e.g. a usual rigid needle shield ("RNS"), an exemplary embodiment of which is shown in Figure 3. The cap D.2 may be integrally formed with the needle shield D.3 or engage the needle shield D.3 by, for example, a friction fit, a snap fit, welding, adhesive, barbs, etc.

In an exemplary embodiment, the package 1 comprises a base element 2 with at least one storage compartment 3 and a needle cap remover 4. The package 1 may be made of a plastic material by a vacuum forming process, injection moulding, etc. The plastic material may be chosen from a material class capable of resisting high strain. For example, the package 1 may be partially manufactured from a polyvinylchloride, polyamide, polyethylene or polypropylene material.

In an exemplary embodiment, the storage compartment 3 may be formed like a depression in the base element 2 and sized and shaped to contain the delivery device D. For example, the storage compartment 3 comprises an outer surface S1 adapted to fit contours of an outer surface S2 of the delivery device D.

In an exemplary embodiment, the needle cap remover 4 is adapted to fix and retain the needle cap D.3. The needle cap remover 4 may be formed as an upstanding projection 4.1 integral with the base element 2. The projection 4.1 may be formed by a vacuum forming process. The projection 4.1 includes a retainer 4.2 adapted to retain the needle cap D.3. In an exemplary embodiment, a recess 4.2.1 is formed on the projection 4.1. The recess 4.2.1 may be adapted to engage a latch D.3.1 on the needle cap D.2, such that when the delivery device D is placed on the needle cap remover 4, the latch D.3.1 engages the recess 4.2.1. Then, when the delivery device D is removed, the needle cap remover 4 retains the needle cap D.2. Since the needle cap D.2 is coupled to the needle shield D.3, removal of the needle cap D.2 will remove the needle shield D.3.

In another exemplary embodiment, the delivery device D may only include the needle shield D.3, and the needle cap remover 4 may be adapted to engage the needle shield D.3. For example, the latch D.3.1 may be formed on the needle shield D.3, and the recess 4.2.1 may engage the latch D.3.1 to remove the needle shield D.3.

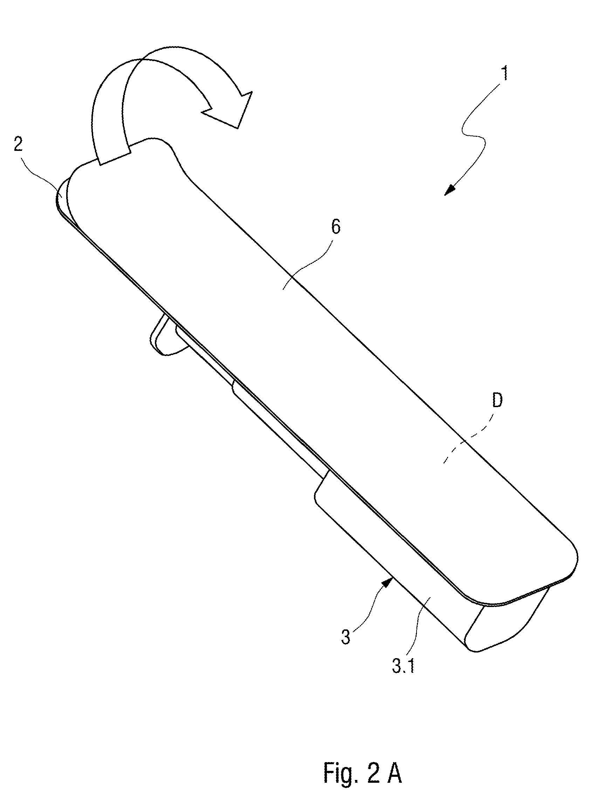

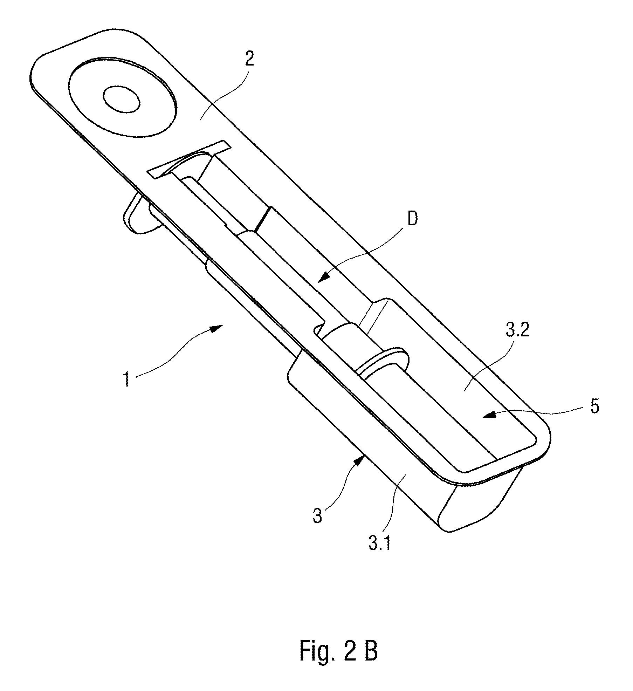

Figures 2A and 2B show an exemplary embodiment of the package 1 in an initial state. Initially, the storage compartment 3 contains the delivery device D in a sterile environment. An opening 5 of the storage compartment 3 is covered by a seal 6. The seal 6 may be coupled to the storage compartment 3 or the base element 2. Adhesive, welding or any other means for coupling the seal 6 to the storage compartment 3 or the base element 2 may be utilized.

Figures 2A shows the package 1 in which the seal 6 is attached, and Figure 2B shows the package 1 in which the seal 6 is removed, exposing the delivery device D in the storage compartment 3. In an exemplary embodiment, the package 1 may be also used for safely storing the used delivery device D. For example, after use, the delivery device D may be re-inserted into the storage compartment 3 for safe disposal. Optionally, the seal 6 may be reapplied to the package 1 to safely enclose the used delivery device 3. Thus, it helps to avoid needle stick injuries with used medical needles and infections that may in particular be transmitted through contact with bodily fluids.

Figure 3 shows the needle cap D.2 retained on the needle cap remover 4 by the latch D.3.1 latching into the recess 4.2.1. Further, the projection 4.1 may be adapted to guide the needle cap D.2 of the delivery device D when the delivery device D is put on the needle cap remover 4, so that the needle cap D.2 may be properly mounted and fixed to the needle cap remover 4.

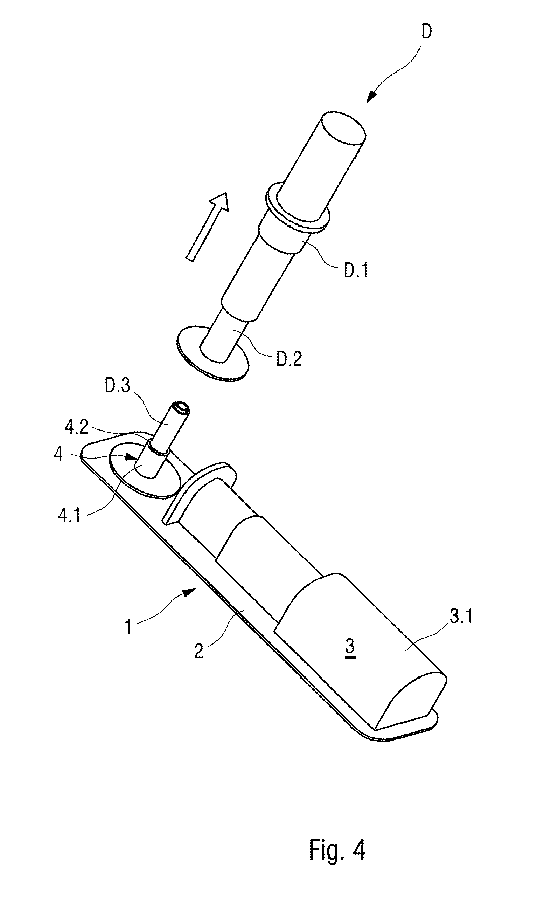

Figure 4 shows an exemplary embodiment of the package 1 after withdrawal of the delivery device D, whereby the needle cap D.2 is removed from the delivery device D and remains on the needle cap remover 4.

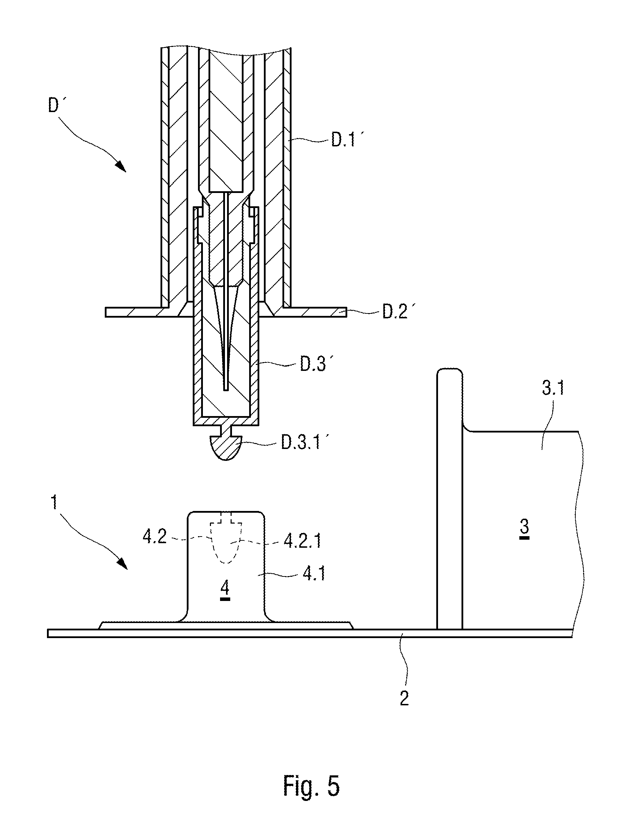

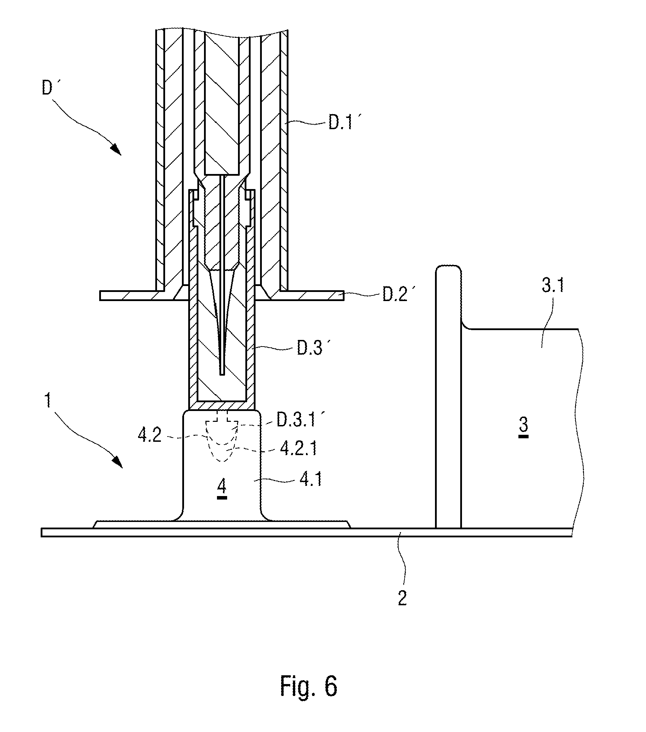

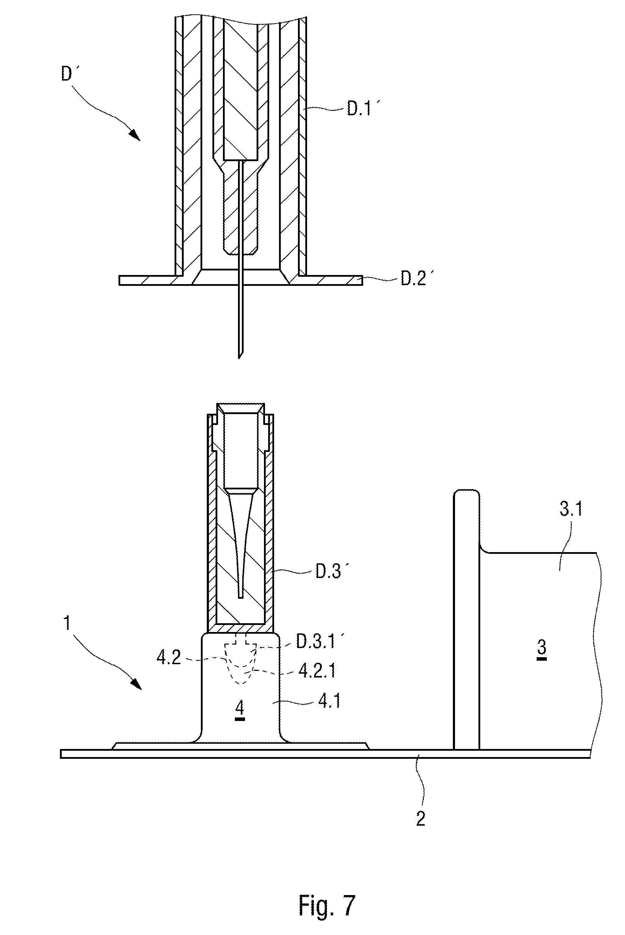

Figures 5 to 7 show an exemplary embodiment of a needle cap remover 4 for removing a needle shield D.3. In this exemplary embodiment, the needle shield D.2 engages the needle cap remover 4. For example, the needle shield D.2 includes the latch D.3.1 which engages a recess 4.2.1 formed in the needle cap remover 4 (shown in Figure 6). The needle shield D.3 is then retained by the interlocking system of the recess 4.2.1 and the latch D.3.1 (shown in figure 7).

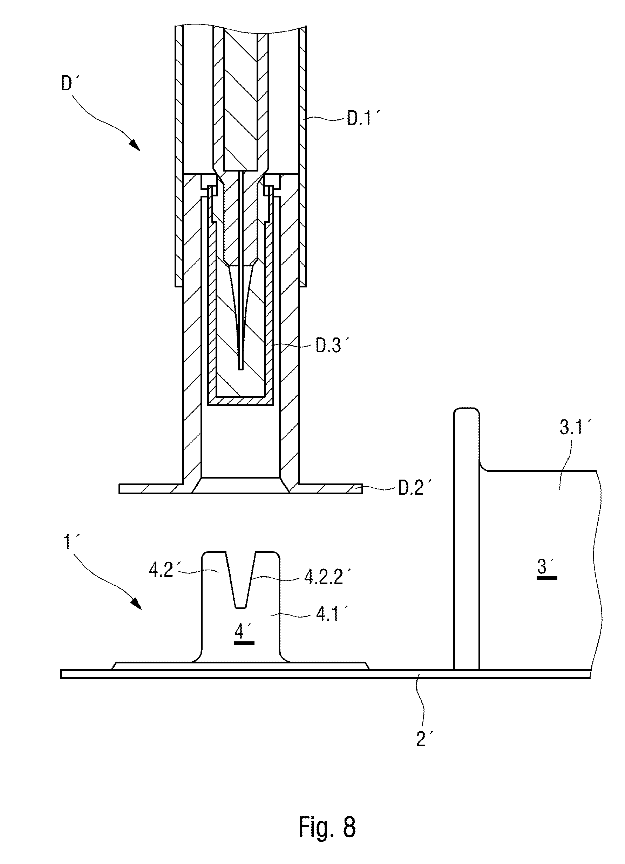

Figure 8 shows another exemplary embodiment of a needle cap remover 4. In this exemplary embodiment, the needle cap remover 4 includes a retainer 4.2 having resilient arms 4.2.2 which deflect when the needle shield D.3 is inserted into the needle cap remover 4. When the delivery device D is removed from the needle cap remover 4, the arms 4.2.2 provide a frictional engagement with the needle shield D.3 to remove it from the delivery device D.

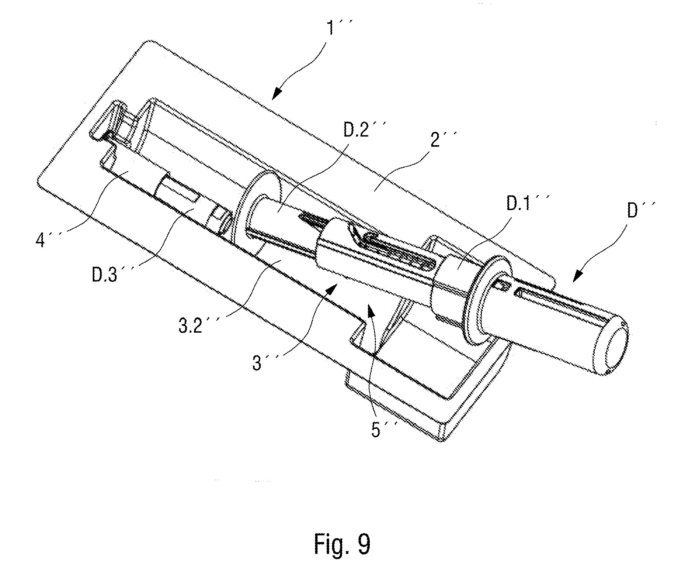

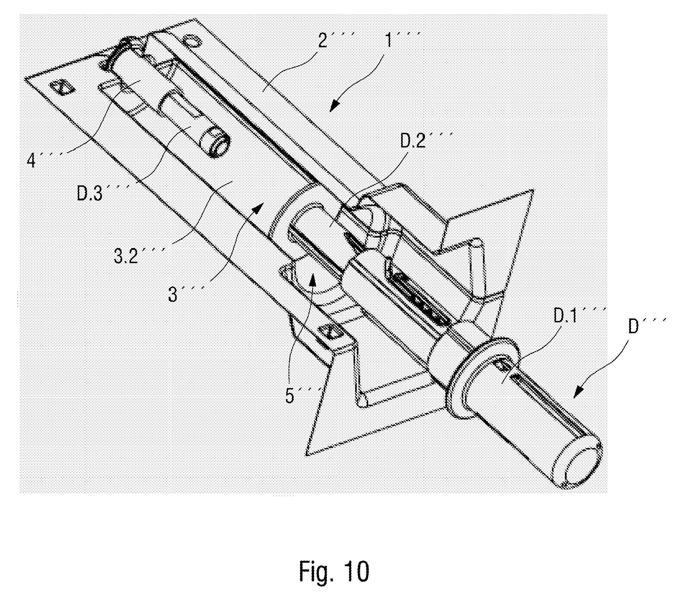

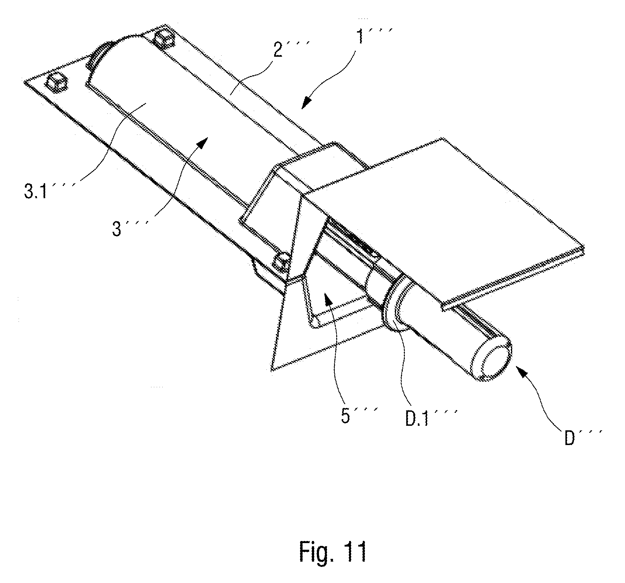

Figures 9 to 11 show further exemplary embodiments of a package 1 with integrated needle cap removers 4. Figure 9 and 10 shows the package 1 with a cut-away to see inner parts of it. The package 1 may cover the delivery device D entirely or partially (e.g., see Figure 11). The needle cap remover 4 is formed in the storage compartment 3. For example, the needle cap remover 4 may be formed in a cavity 3.2 in the storage compartment 3 and be adapted to engage the needle cap D.2 and/or needle shield D.3 when the delivery device D is packaged.

In this exemplary embodiment, the needle cap remover 4 may comprise a 4.2.1 adapted to engage a latch D.3.1 on the needle cap D.2 or needle shield D.3. Thus, when the delivery device D is being removed from the package 1, the needle cap D.2 and/or the needle shield D.3 may be retained by the needle cap remover 4. After use the delivery device D may be re-inserted into the storage compartment 3, so that the delivery device engages the needle cap D.2 and/or the needle shield D.3 retained in the needle cap remover 4. Thus, the package 1 may be used as a disposal container for used delivery devices D.

Those of skill in the art will understand that modifications (additions and/or removals) of various components of the apparatuses, methods and/or systems and embodiments described herein may be made without departing from the full scope and spirit of the present invention, which encompass such modifications and any and all equivalents thereof.

## Claims

1. A package (1) for a medicament delivery device (D) having a needle shield (D.3) covering a needle, the package (1) comprising:
a base element (2) including a storage compartment (3) adapted to contain the delivery device (D);
a needle cap remover (4) formed on the base element (2), the needle cap remover (4) adapted to engage the needle shield (D.3).

2. The package according to claim 1, wherein the needle cap remover (4) is an upstanding projection (4.1).

3. The package according to claim 1, wherein the needle cap remover (4) is in the storage compartment (3).

4. The package according to any one of the preceding claims, wherein the needle cap remover (4) comprises at least a retainer (4.2) adapted to retain the needle shield (D.3).

5. The package according to claim 4, wherein the retainer (4.2) is includes resilient arms (4.2.2) adapted to deflect when the needle shield (D.3) is inserted into the retainer (4.2).

6. The package according to claim 5, wherein the resilient arms (4.2.2) frictionally engage the needle shield (D.3).

7. The package according to claim 4, wherein the retainer (4.2) is a recess (4.2.1) adapted to engage a latch (D.3.1) on the needle shield (D.3).

8. The package according to any one of the preceding claims, further comprising:
a seal (6) covering the storage compartment (3).
